# EUROPEAN PATENT SPECIFICATION

(11) **EP 4 061 970 B1**
(45) Date of publication and mention of the grant of the patent: **25.12.2024**
(21) Application number: 20815963.2
(22) Date of filing: 20.11.2020
(51) Int. Cl.: C14C 1/06, C14C 3/28, C14C 3/06

(54) **PROCESS FOR DEHAIRING AND LIMING OF HIDES, SKINS OR PELTS**
VERFAHREN ZUM ENTHAAREN UND KÄLKEN VON TIERHÄUTEN UND TIERBLÖSSEN
PROCÉDÈ D'ÉPILAGE ET DE PELANAGE DES PEAUX ET DES PEAUX EN TRIPE

(30) Priority: 22.11.2019 NL 2024293
(43) Date of publication of application: 28.09.2022
(73) Proprietor: Stahl International B.V., 5145 PE Waalwijk (NL)
(72) Inventor: VAN DER HAM, Andreas Gerardus Josephus Maria, 5145 PE Waalwijk (NL)
(74) Representative: V.O.
(86) International application number: PCT/NL2020/050731
(87) International publication number: WO 2021/101381

(56) References cited:
- US-A- 1 765 199
- US-A- 4 548 608
- US-A1- 2005 102 761
- SIRVAITYTE J ET AL: "Immunization Action of Sodium Silicate on Hair: Part 2 Hair-save Process Based on Lime Substitution by Sodium Silicate", JOURNAL OF THE SOCIETY OF LEATHER TECHNOLOGISTS AND CHEMISTS, SOCIETY OF LEATHER TECHNOLOGISTS AND CHEMISTS, GB, vol. 99, 1 January 2015 (2015-01-01), pages 231 - 237, XP009521521, ISSN: 0144-0322

## Description

The present invention relates to a novel process for substantially lime free dehairing in skins/hides/pelts to obtain dehaired skins/hides/pelts of good quality. The total or at least substantial elimination of lime (calcium hydroxide) in the dehairing process leads to reduced TDS (total dissolved solids), reduced TSS (total suspended solids), COD (chemical oxygen demand), an improved ratio in BOD₅/COD (BOD₅ is biological oxygen demand, measured in five days), a reduced total nitrogen content and reduced sulphide concentration in the effluent. The TDS, BOD₅ and COD are used as measures for the environmental impact of the effluent. The invention is set out in the appended set of claims

It is well known that in the leather manufacturing process, pre-treatments for raw skins/hides/pelts shall be done before tanning and crusting. Dehairing, often called liming due to the calcium hydroxide that is frequently used, is one of the pre-treatments and a key step to remove hair and unwanted proteins from the skins/hides/pelts and to open up the collagen fibre structure. The leather manufacturing process generally consists of the following steps: soaking (dirt removal and re-hydration); dehairing (removal of hair, traditionally part of the liming process); liming (removal of hair and degradation of fats and proteins as well as swelling of the collagen structure, so called opening up); fleshing (removal of fatty tissue); splitting (horizontal cutting into grain split and flesh split); deliming (releasing lime, degraded proteins/fat and reducing pH); bating (removal of non-structural proteins, scut removal and fibre opening); pickling (lowering of pH value to around 3) and tanning (stabilization of the skin or hide matrix).

During the dehairing step the skins/hides/pelts are normally treated with an alkaline and a sulphide solution which aims at breaking the hair structure at its weakest point, the root of the hairs, by reduction of sulphur-sulphur bonds in the keratin. Lime is normally used as the vital alkaline agent to achieve graduate and controlled swelling effect. Subsequently, the lime must be removed to get the pelts prepared for further processing. Deliming is about removing calcium hydroxide from the skins/hides/pelts, including surfaces and inner parts thereof, and lowering pH value of skins/hides/pelts until around 7. A lower pH means the swelling is reduced, which causes the degraded protein/fat from inside the hide to flow into the float. The main deliming is carried out with acids, acid salts and amides, e.g. phthalic acid, sulfophthalic acid, boric acid, mixture of dicarboxylic acids, polysuccinimide, esters and ammonium salts.

The current method of liming of skins/hides/pelts has serious disadvantages in industrial practice. In the whole leathering manufacturing process, the beamhouse process (i.e. the process for preparing the hides for tanning) accounts for 80% of organic waste in effluent and sludges. Among these, around 70% comes from the liming process. In traditional liming process, calcium hydroxide (lime) is used in combination with sodium sulphide and this liming process usually takes from 18 hours to 24 hours depending upon the method employed. Besides solid waste from hair pulp, lime and organic matter, which make a sludge, also air pollution due to release of hydrogen sulphide is often occurring. An additional source of waste is the deliming process that usually is applied subsequent to the liming process. Deliming agents used in leather industry are mainly based on ammonium salts, which are widely accepted in leather industry. Ammonium salts used for deliming purpose, react with calcium hydroxide which is physically and chemically bonded on the limed skins/hides/pelts. Ammonium chloride usage will result in calcium chloride and ammonia and ammonium sulphate usage will result in calcium sulphate and ammonia. The ammonia that is released in this reaction results in undesired exposure to workers and in a necessity to remove ammonia from tannery effluent, which increases the cost and duration of wastewater treatment.

Dehairing processes without using lime have been described, even in a text book like 'Leather Technician's Handbook' by J.H. Sharphouse, 1989, ISBN 0950228516, page 111, where sodium hydroxide is discussed as such an alternative and it is mentioned that large amount of sodium hydroxide would be needed, which would give a risk for excessive swelling and protein hydrolysis.

Dehairing processes without lime using enzymes have also been described.

US 7198647B2 describes a dehairing process using animal and/or herbal enzymes.

US 6957554B2 describes a process for making skins/hides/pelts by dehairing and fibre opening employing enzyme and optionally a silicate salt. However, the dehairing is done by applying a paste on the skins/hides/pelts, which is a cumbersome method.

US 9267182B2 and US 9856540B2 describe a method for the processing of skins/hides/pelts into leather, comprising enzymatic treatment of the skins/hides/pelts with bacterial glutamyl endopeptidase.

US 6957554B2 essentially discloses a process, which involves a step of dehairing using proteolytic enzymes optionally assisted with silicate salt, followed by a second step of fibre opening using silicate salt. The process does not provide any possibility of carrying out dehairing as well as fibre opening in the first step itself even if silicate is used therein because of the adjustment of conditionalities therein. Moreover, silicate is associated with a limitation of exhibiting its activity when used in paste form.

The use of sodium silicate in the dehairing step is described in Journal of the Society of Leather Technologists and Chemists, volume 99, 2015, pages 231-237. In this article also additional sodium hydroxide was used, but in a separate consecutive process step, and an additional process step is undesired.

US 4548608 discloses an aqueous composition for removing hair from full hair whole carcasses, skins, hides and pelts. The aqueous composition as used for dehairing however contains only a minor amount of sodium silicate compared to alkaline.

A major advantage of using sodium silicate is that sodium silicate readily dissolves in water, whereas the traditional calcium hydroxide results in a large amount of suspended solids in the float because it dissolves poorly in water. However, using sodium silicate yields less hide swelling than using calcium hydroxide, which means that sodium silicate does not result in sufficient dermal opening.

Therefore, the object of the present invention is to provide a substantially lime-free process for dehairing skins/hides/pelt and that provides skins/hides/pelts of good quality.

It is a further object of the present invention to provide dehairing and liming agents for skins/hides/pelts that lead to less solid waste than the currently used dehairing and liming agents, that lead to less polluted wastewater, that lead to similar or improved quality of crust and leather and yet are very effective dehairing and liming agents.

According to the present invention a process for dehairing or loosening of hairs and liming of hides, skins or pelts is provided, comprising treating the hides, skins or pelts substantially in the absence of calcium hydroxide in an aqueous solution simultaneously with a metal silicate and an alkaline.

It has surprisingly been found that using a metal silicate in combination with an alkaline in a substantially lime-free dehairing/liming step results in a better quality of crust and final leather than would be obtained when using either calcium hydroxide on its own or a sodium silicate on its own as sole replacement for calcium hydroxide.

The combination of alkaline and metal silicate of the present invention mainly acts as an dehairing auxiliary and a liming agent. This means that generally in order to achieve complete and sufficient dehairing using these substances usually additional compounds are needed such as a protein disulphide reducing agent or an enzyme to assist in the breaking or softening of the hair roots.

In the context of the present invention substantially lime free, or substantially in the absence of lime means that the amount of lime (calcium hydroxide) present in the aqueous composition is less than 0.2 weight%, preferably less than 0.1 weight% where the weight percentage is the percentage of calcium hydroxide based on the weight of the aqueous treating composition. The amount of lime is then generally less than 0.5 weight%, preferably less than 0.2 weight%, where the weight percentage is the percentage based on the weight of the salted pelt.

An advantage of the process of the present invention is that a much reduced TSS (total suspended solids) is achieved. In general the TSS level is reduced by at least 50 % by using the dehairing and liming agents of the present invention compared to the use of lime

A further advantage of the process of the present invention is that a reduced TDS (total dissolved solids), an improved ratio in BOD₅/COD, a reduced sulphide concentration and sometimes also a reduced COD in the effluent is achieved.

TDS (total dissolved solids) is measured via conductivity. Dissolved ionized solids, such as salts and minerals, increase the electrical conductivity of a solution. Because it is a volume measure of ionized solids, electrical conductivity can be used to determine TDS. Dissolved organic solids, such as sugar, and microscopic solid particles, such as colloids, do not significantly affect the conductivity of a solution, and are not taken into account in the TDS. A lower TDS value, expressed in mg per kg of skin/pelt, is desired.

COD (chemical oxygen demand) is measured by reacting the oxidizable substance with sulphuric acid and potassium dichromate solution in the presence of silver sulphate as a catalyst. Chloride is masked by mercury sulphate. The value is derived from the intensity of the green coloration of Cr³⁺. The method is according to ISO 15705. A lower COD value, expressed in mg per kg of skin/pelt, is desired.

BOD₅ (biological oxygen demand) is the 5-day biochemical oxygen demand, where nitrification is inhibited by 5 mg/L allylthiourea. The dissolved oxygen is analysed in an alkaline solution with a pyrocatechol derivative in the presence of Fe²⁺, under which conditions a red dye is formed which is measured in a photospectrometer. The method is according to EN 1899-1. The ratio BOD₅/COD is calculated from the measured values of COD and BOD₅. A BOD₅/COD ratio of between 0.3 and 0.6 is seen as optimum, because this means that the effluent is better biodegradable in a wastewater treatment facility.

TSS (total suspended solids) is measured by weight. A glass fiber filter disc, of which the empty weight was determined, is used as a filter in a filtering flask. A defined sample volume of effluent is filtered over this filter and the residue, on the same fiber filter disc, is dried to a constant weight in an oven at 105°C. The weight difference is the Total Suspended Solids. According to the volume used, the suspended solids can be expressed as mg/L, which can be recalculated to mg per kg of skin/pelt. The method is according to ISO 11923:1997. A lower TSS value, expressed in mg per kg of skin/pelt, is desired.

A further advantage of the present invention is that there is less need for surface active agents in the dehairing/liming step itself, as no lipid or grease balls are observed, which are being formed in the classic method using calcium hydroxide and which are then dissolved by the use of surface active agents.

A further advantage of the present invention is that the subsequent deliming step in the beamhouse process does not result in a calcium salt in the tannery effluent, which is the case when calcium hydroxide is used in the dehairing/liming step. Instead other salts are being formed upon the addition of deliming agents, which is advantageous because these other salts, such as sodium salts, potassium salts, are generally better soluble in water and yield thus less solid waste.

### Beamhouse process

In the process steps described below percentages are based on weight of skins/hides/pelts, unless otherwise indicated.

As a substrate to be dehaired according to the invention there may be employed any hides, skins or pelts as conventionally processed in the beamhouse. There may e.g. be mentioned pelts, hides or skins from sheep, goat, swine, cattle and other animals (e.g. horse, colt, doe, deer, ostrich, poultry, dromedary, camel and camel-like animals - e.g. lama or alpaca), and wooled skins (mainly from sheep or goat) and furskins. The substrate can be treated with salt (NaCl) to preserve them and to prevent rotting.

### Soaking

When the salted skins/hides/pelts enter the beamhouse they are subjected to a dirt soak to remove salt and dirt. The duration can be adapted to the process of the beamhouse and may vary from 1 hour to 12 hours. The dirt soak is normally followed by a longer soak which is conventionally between 8 and 72 hours. This soak serves to rehydrate the skins/hides/pelts and starts the opening of the fibre structure. These soaking steps are generally carried out in paddle, drum or mixer as mechanical agitation accelerates the soaking process.

### Dehairing

Dehairing is conventionally performed with sulphide and lime (calcium hydroxide) as described above.

The present invention however provides a more efficient and more environmental-friendly dehairing process. In the dehairing process of the present invention a metal silicate and an alkaline are used to treat the soaked skins/hides/pelts, without the addition of substantial amounts of calcium hydroxide. By adding the mix of metal silicate and alkaline in this dehairing step the pH is increased and immunization (transformation of the sulphur-sulphur bonds in the hair) is achieved.

Silicates are a family of anions consisting of silicon and oxygen usually with the general formula [SiO₄₋ₓ ^{(4-2x)-}] wherein 0 ≤ x < 2. The family includes orthosilicate wherein x = 0, metasilicate wherein x = 1 and pyrosilicate wherein x = 0.5 and n = 2. The term silicate usually also covers any salt of such anions such as sodium metasilicate or any ester thereof such as tetramethyl orthosilicate. Any metal silicate can be used according to the invention, particularly those silicates based on sodium. Potassium and/or lithium based silicates can be used as well as long as their solubility is similar as that of sodium silicate. Suitable metal silicates to be used in the present invention include sodium metasilicate (also known as water glass when dissolved in water), sodium orthosilicate, potassium silicate, lithium silicate, either individually or in any combination.

Alkaline to be used in the present invention should preferably be strong alkaline that is able to increase the pH of the bath to above 12. Suitable alkalines include metal hydroxide such as sodium hydroxide, potassium hydroxide and lithium hydroxide but excludes calcium hydroxide.

The conditions under which the treatment with a metal silicate and an alkaline is performed can be varied according to the specific metal silicate and ratio of metal silicate versus alkaline chosen. Some of the parameters that can be varied are described below. Either the parameter may be varied alone or any combination of the parameters may be varied at the same time.

Generally the weight ratio between the metal silicate and the alkaline to be used in the dehairing step of the present invention is between 90/10 and 75/25, preferably between 90/10 and 80/20, most preferably between 85/15 and 80/20 where the ratio is based on weight of both solid materials and where the major component is the metal silicate. If the ratio metal silicate and alkaline are within these ranges the quality of the obtained leather is superior.

The amount of total metal silicate and alkaline dehairing agent is in the range of 0.2% to 8%, preferably from 0.5% to 4%, more preferably from 1% to 2.5% per kg of hide, skin or pelt.

Optionally, the treatment of the soaked skins/hides/pelts with metal silicate and alkaline according to the invention is preceded by a treatment with any of an enzyme, reducing agent, a mercaptan product, a probiotic auxiliary, which can also function as a dispersing agent. Treatment with enzyme will result in a reduced need for sulphide components lateron. Treatment with reducing agent, such as a mercaptan product, will assist in breaking or softening the hair roots.

The treatment of the soaked skins/hides/pelts with metal silicate and alkaline is preferably done in the presence of a protein disulphide reducing agent, such as hydrosulphide and/or sulphide. In the context of the present invention hydrosulphide and/or sulphide are preferred protein disulphide reducing agents. The protein disulphide reducing agent will make sure that the hair is released from the hair roots by breaking the sulphur-sulphur bonds in the hair/roots that have not been immunized. The sulphide salt used may be selected from sodium sulphide, sodium hydrosulphide, either individually or in combination. Preferably, the hydrosulphide and sulphide are sodium hydrosulphide and sodium sulphide.

The metal silicate and alkaline dehairing treatment time may be adjusted such that there is a sufficient hair removal and very limited to no grain damage. In an embodiment of the present invention the treatment time is between 0.1 and 5 hours, preferably between 0.1 and 2 hours.

The float composition can be optimized and varied as suitable. The skilled person will know how to make such variations. The float composition is the contents of the drum besides the skins/hides/pelts, which is thus mostly water with the various chemicals and agents that have been added, but also organic materials that originated from the skins/hides/pelts. Generally, the float composition is based on water. The float may also contain wetting agents, degreasing agents and biocides.

The dehairing treatment is generally carried out in connection with mechanical action, e.g using a paddle, drum or mixer as mechanical agitation to accelerate the process. As a guideline, hides are treated in a drum with a float of 50% to 400%, preferably from 50% to 200% per kg of hide, skin or pelt. The treatment can be performed in the temperature range of 5°C to 30°C, preferably in the range of 10°C to 25°C, more preferably in the range of 15°C to 20°C.

### Liming

The liming step is the conventional final dehairing step in the beamhouse process, which generally uses sulphide to reduce the disulphide bridges in the keratin molecules, and calcium hydroxide to loosen the collagen structure and releases interfibrillary noncollagenous proteins. The last remnants of hair are removed hereby.

In the context of the present invention this step is performed substantially in the absence of calcium hydroxide hence without the substantial addition of calcium hydroxide. Instead, metal silicate and alkaline are employed as liming agent. Their addition will achieve fibre opening and removal of non-structural protein. The skilled person in the art will know how to optimize the amount of liming agent. The amount of total metal silicate and alkaline liming agent is in the range of 0.2% to 8%, preferably from 0.5% to 4%, more preferably from 1% to 2.5% per kg of hide, skin or pelt. The weight ratio of metal silicate and strong alkaline is in the range of 90/10 to 75/25, more preferably in the range of 90/10 to 80/20, where the ratio is based on weight of both solid materials and where the major component is the metal silicate.

So in the process of the present invention all steps involved in the dehairing and liming of hides, skins or pelts are carried out substantially in the absence of calcium hydroxide meaning that the amount of calcium hydroxide present in the dehairing/liming composition is less than 0.2 % by weight based on the total weight of the composition.

The treatment with metal silicate and alkaline in the liming step can be accompanied by a sulphide treatment or a treatment with an alternative protein disulphide-reducing compound in order to release the hairs even more efficiently. In the following, it should be understood that when the term sulphide is used it includes alternative protein disulphide reducing compounds unless stated otherwise. The skilled person will know which sulphides are suitable in the beamhouse process, some examples are Na₂S, CaS, As₂S₃ and NaHS, as well as other salts of the same. Alternative protein disulphide reducing compounds could be salts of thioglycolic acid as well as other thiols (mercaptans) or enzymes capable of catalysing the rearrangement of disulphide bonds in protein. The skilled person in the art will know how to optimize the amount of sulphide. In a preferred embodiment, the amount of sulphide is the range of 0.05% to 4%, preferably from 0.1% to 2%, more preferably from 0.2% to 1.5% per kg of hide, skin or pelt.

### Deliming

In the beamhouse process, deliming is performed after the liming process, to remove the liming agent from the pelts and to reduce the pH to between 7 and 8.5. The reduction in pH causes the swelling to reverse, which causes an outflow of degraded protein/fat to the float. This is important to prepare the pelt for the remaining part of the beamhouse process.

### Pickling and Tanning

These processes are the remaining steps in the beamhouse process and will not be affected by the modified procedures described above. Some beamhouse processes also include a bating step, which serves to remove additional proteins, this is however an optional step in the beamhouse process of the present invention. The skilled person in the art will know how to conduct these steps.

The dehairing and liming process of the present invention is exemplified below in detail and generally comprises the following steps:
i) Optionally, the soaked skins/hides/pelts are treated with 0.05-0.5% of enzymes, 0.01-0.1% of probiotic auxiliaries, 0.05-0.3% of reducing agent and 0.10-1.0% of a mercaptan product at a temperature of 10-25°C in the presence of 50-150% of water, preferably under stirring condition.
ii) A metal silicate and an alkaline is added and the first treatment is executed for a period of between 10 minutes and 1 hour. The amount of metal silicate and an alkaline combined is in the range of 0.2% to 8%, preferably from 0.5% to 4%, more preferably from 1% to 2.5% per kg of hide, skin or pelt. The ratio of metal silicate and alkaline is preferably in the range of 90/10 to 75/25, more preferably in the range of 90/10 to 80/20, where the ratio is based on weight of both solid materials and where the major component is the metal silicate. Optionally, a probiotic agent, degreasing agent and/or wetting agent is present. In this step the pH is increased and if hair saving (by immunization) is desired then the treatment takes long, about 1 hour. If hair is burned then the treatment is generally shorter, about 5 minutes since immunization of the hairs is not needed then.
iii) Next, one or more protein disulphide reducing agents are added, such as 0.2-3.5% of sodium hydrosulphide and/or 0.1-3.0% sodium sulphide, and the treatment is continued for between 2 hours and 8 hours, after or during which the immunized hair is removed by filtration.
iv) Next, water, metal silicate and an alkaline are added and optionally a further amount of one or more protein disulphide reducing agents, such as 0.1-1.0% of sodium sulphide is added. The treatment is continued for between 3 hours to 20 hours. The amount of metal silicate and an alkaline combined is in the range of 0.2% to 8%, preferably from 0.5% to 4%, more preferably from 1% to 2.5% per kg of hide, skin or pelt. The actual amount of metal silicate and alkaline used in this step is generally lower than the amount used in step (ii). The ratio of metal silicate and alkaline is preferably in the range of 90/10 to 75/25, more preferably in the range of 90/10 to 80/20, where the ratio is based on weight of both solid materials and where the major component is the metal silicate.
(v) Optionally, 0.1-1.0% of an oxidizer is added to remove residual sulphide present in the float. After running for between 10 to 120 minutes, the skins/hides/pelts are removed from the drums and fleshed and splitted.

Thereafter, using conventional methods, crust is obtained after deliming (actually neutralization step), bating, pickling, chrome tanning, retanning, neutralization, dyeing, and fatliquoring.

The crust or leather obtained by using the process of the present invention is of very good quality. The feel of the leather is more full and more tight, the leather is more open and the brightness of the leather is higher, compared to leather obtained using either calcium hydroxide or only metal silicate as a dehairing and liming agent. These properties are visually and tactilely assessed.

The substantial or even total elimination of lime (calcium hydroxide) in the dehairing process leads to reduced TDS, TSS, an improved ratio in BOD₅/COD, a reduced total nitrogen content, reduced sulphide concentration and in some cases to a reduced COD in the effluent. The TDS, TSS, BOD₅, COD, total nitrogen and sulphide concentration are used as measures for the environmental impact of the effluent. A reduced TSS means that much less solid waste is generated by the process, and by using the dehairing and liming process of the present invention the resulting TSS is considerably lower than obtained via the conventional process using lime. The resulting TSS of the process of the present invention is generally less than half of what is normally obtained as TSS via the conventional process using lime. A reduced TDS, reduced COD, improved ratio in BOD₅/COD and reduced sulphide concentration in the effluent means that the effluent is cleaner and better biodegradable. And a reduced total nitrogen content indicates that less protein degradation has occurred during the dehairing steps. A lower nitrogen content in the effluent is also beneficial for the biodegradability because tannery effluent usually contains too much nitrogen compared to carbon as the optimum ratio between nitrogen and carbon is about 1 to 100 and lower nitrogen content in the effluent means that the ratio will come closer to the 1 to 100 ratio. A ratio of between 0.3 and 0.6 is seen as optimum for the BOD₅/COD ratio, because this means that the effluent is better biodegradable in wastewater treatment facility.

The following examples serve to illustrate the invention, but they are not intended to limit it thereto:

### Example 1

Italian cattle hide that has been soaked in conventional way was used for the application trial. The dehairing and liming process comprised the following steps:
(i) Soaked pelt was pre-dehaired in a drum with 50% (% is weight percentage based on the weight of the salted pelt) of water at 25°C, with 0.15% of enzymes (Bemanol RS-300; obtainable from Stahl Europe BV), 0.03% of probiotic auxiliaries (ProSoak; obtainable from Stahl Europe BV), 0.15% of reducing agent (Bemanol RS-240; obtainable from Stahl Europe BV) and 0.60% of a mercaptan product (Mollescal MF; obtainable from Stahl Europe BV) and 8.5% of ice water, followed by adding 0.15% of sodium sulphide to start the further dehairing process. The mixture was ran for 20 minutes.
(ii) A probiotic auxiliary (0.05% of ProDegreaze; obtainable from Stahl Europe BV), 8.5% of ice and a mixture of 1.64% of sodium metasilicate and 0.16% sodium hydroxide (ratio 90/10) were added. The mixture was ran for 30 minutes and stood still for 15 minutes thereafter.
(iii) 0.85% of sodium hydrosulphide, 17.0% of ice water (as cooling) and 0.50% of sodium sulphide were added. After running for 120 minutes, the immunized hair was removed from the bath by filtration. After running 10 minutes and stopping 5 minutes consecutively in 12 cycles for 180 minutes, dehairing was considered finished.
(iv) 80% of water, 0.30% of sodium sulphide and a mixture of solids of 1.26% sodium metasilicate and 0.14% sodium hydroxide (ratio 90/10) were added. The mixture was left overnight on a program, running 5 minutes and stopping 25 minutes consecutively.
(v) Next, 0.70% of an oxidizer (Bemanol RS-230; obtainable from Stahl Europe BV) was added to remove residual sulfide present in the float. After running for 45 minutes, the pelts were removed from the drums and fleshed and splitted.

Thereafter, using conventional methods, crust was obtained after deliming (actually neutralization step), bating, pickling, chrome tanning, retanning, neutralization, dyeing and fatliquoring.

### Example 2

Italian cattle hide which has been soaked in conventional way was used for the application trial. The dehairing process comprised the following steps:
(i) Soaked pelt was pre-dehaired in the drum with 50% (% is weight percentage based on the weight of the salted pelt) of water at 30°C, with 0.15% of enzymes (Bemanol RS-300; obtainable from Stahl Europe BV), 0.03% of probiotic auxiliaries (ProSoak; obtainable from Stahl Europe BV), 0.15% of reducing agent (Bemanol RS-240; obtainable from Stahl Europe BV) and 0.60% of a mercaptan product (Mollescal MF; obtainable from Stahl Europe BV), followed by adding 0.15% of sodium sulphide to start the further dehairing process. The mixture was ran for 20 minutes.
(ii) A probiotic auxiliary (0.05% of ProDegreaze; obtainable from Stahl Europe BV) and a mixture of 1.53% of sodium metasilicate and 0.27% sodium hydroxide (ratio 85/15) were added. The mixture was ran for 30 minutes and stood still for 15 minutes thereafter.
(iii) 0.85% of sodium hydrosulphide and 0.50% of sodium sulphide were added and the drum was cooled on the outside with running tap water. After running for 120 minutes, the immunized hair was removed from the bath by filtration.
(iv) After running 10 minutes and stopping 5 minutes consecutively in 12 cycles for 180 minutes, dehairing was considered finished and 80% of water, a mixture of solids of 1.19% sodium metasilicate and 0.21% sodium hydroxide (ratio 85/15) and 0.30% of sodium sulphide were added.
(v) The mixture was left overnight on a program, running 5 minutes and stopping 25 minutes consecutively. Next, 0.70% of an oxidizer (Bemanol RS-230; obtainable from Stahl Europe BV) was added to remove residual sulfide present in the float. After running for 45 minutes, the pelts were removed from the drums and fleshed and splitted.

Thereafter, using conventional methods, crust was obtained after deliming (actually neutralization step), bating, pickling, chrome tanning, retanning, neutralization, dyeing and fatliquoring.

### Comparative Example 3

Italian cattle hide which has been soaked in conventional way was used for the application trial. The dehairing process comprised the following steps:
(i) Soaked pelt was pre-dehaired in the drum with 50% (% is weight percentage based on the weight of the salted pelt) of water at 25°C, with 0.15% of enzymes (Bemanol RS-300; obtainable from Stahl Europe BV), 0.03% of probiotic auxiliaries (ProSoak; obtainable from Stahl Europe BV), 0.15% of reducing agent (Bemanol RS-240; obtainable from Stahl Europe BV) and 0.60% of a mercaptan product (Mollescal MF; obtainable from Stahl Europe BV) and 8.0% of ice, followed by adding 0.15% of sodium sulphide to start the further dehairing process. The mixture was ran for 20 minutes.
(ii) A probiotic auxiliary (0.05% of ProDegreaze; obtainable from Stahl Europe BV), 8.0% of ice and a mixture of 1.44% of sodium metasilicate and 0.36% sodium hydroxide (ratio 80/20) were added. The mixture was ran for 30 minutes and stood still for 15 minutes thereafter.
(iii) 0.85% of sodium hydrosulphide, 16.0% of ice water (as cooling) and 0.50% of sodium sulphide were added. After running for 120 minutes, the immunized hair was removed from the bath by filtration. After running 10 minutes and stopping 5 minutes consecutively in 12 cycles for 180 minutes, dehairing was considered finished.
(iv) 80% of water and a mixture of solids of 1.12% sodium metasilicate and 0.28% sodium hydroxide (ratio 80/20), 0.30% of sodium sulphide were added. The mixture was left overnight on a program, running 5 minutes and stopping 25 minutes consecutively.
(v) Next, 0.70% of an oxidizer (Bemanol RS-230; obtainable from Stahl Europe BV) was added to remove residual sulfide present in the float. After running for 45 minutes, the pelts were removed from the drums and fleshed and splitted.

Thereafter, using conventional methods, crust was obtained after deliming (actually neutralization step), bating, pickling, chrome tanning, retanning, neutralization, dyeing and fatliquoring.

### Example 4

The same process as described in comparative Example 3 was followed, except that in step ii) a mixture of solids of 1.35% sodium metasilicate and 0.45% sodium hydroxide (ratio 75/25) was used instead of a mixture of solids of 1.44% sodium metasilicate and 0.36% sodium hydroxide (ratio 80/20), and in step iv) a mixture of solids of 1.05% sodium metasilicate and 0.35% sodium hydroxide (ratio 75/25) was used instead of a mixture of solids of 1.12% sodium metasilicate and 0.28% sodium hydroxide (ratio 80/20).

### Example 5

The same process as described in comparative Example 3 was followed, except that in step ii) a mixture of solids of 1.26% sodium metasilicate and 0.54% sodium hydroxide (ratio 70/30) was used instead of a mixture of solids of 1.44% sodium metasilicate and 0.36% sodium hydroxide (ratio 80/20), and in step iv) a mixture of solids of 0.98% sodium metasilicate and 0.42% sodium hydroxide (ratio 70/30) was used instead of a mixture of solids of 1.12% sodium metasilicate and 0.28% sodium hydroxide (ratio 80/20).

### Comparative Example 6 with calcium hydroxide

Italian cattle hide which has been soaked in conventional way was used for the application trial. The liming/dehairing process comprises the following steps:
(i) Soaked pelt was pre-dehaired in the drum with 50% (% is weight percentage based on the weight of the salted pelt) of water at 30°C, with 0.15% of enzymes (Bemanol RS-300; obtainable from Stahl Europe BV), 0.03% of probiotic auxiliaries (ProSoak; obtainable from Stahl Europe BV), 0.15% of reducing agent (Bemanol RS-240; obtainable from Stahl Europe BV) and 0.60% of a mercaptan product (Mollescal MF; obtainable from Stahl Europe BV) and 9.0% of ice water, followed by adding 0.15% of sodium sulphide to start the further dehairing process. The mixture was ran for 20 minutes.
(ii) A probiotic auxiliary (0.05% of ProDegreaze; obtainable from Stahl Europe BV), 9.0% of ice and 1.80% of calcium hydroxide were added to increase the pH of the float and initiate immunization. The purpose of this step is to immunize the hairs to facilitate the hair saving process. The mixture was ran for 30 minutes and stood still for 15 minutes thereafter.
(iii) 0.85% of sodium hydrosulphide, 18.0% of ice water and 0.50% of sodium sulphide was added. After running for 120 minutes, the immunized hair was removed from the bath by filtration. Next, 0.02% of Eusapon EM (a wetting agent obtainable from Stahl Europe BV) and 0.10% of Tergolix W-01 (a wetting agent obtainable from Stahl Europe BV) were added and then after running 10 minutes and stopping 5 minutes consecutively in 12 cycles for 180 minutes, dehairing was considered finished
(iv) Liming was initiated by adding 80% of water, 1.40% of calcium hydroxide, 0.30% of sodium sulphide and 0.02% of Eusapon EM (a wetting agent obtainable from Stahl Europe BV). The liming was left overnight on a program, running 5 minutes and stopping 25 minutes consecutively.
(v) Next, 0.70% of an oxidizer (Bemanol RS-230; obtainable from Stahl Europe BV) was added to remove residual sulfide present in the float. After running for 45 minutes, the pelts were removed from the drums and fleshed and splitted.

Thereafter, using conventional methods, crust was obtained after deliming, bating, pickling, chrome tanning, retanning, neutralization, dyeing and fatliquoring.

### Comparative Example 7 with sodium silicate

Dutch cattle hide which has been soaked in conventional way was used for the application trial. The liming/dehairing process comprises the following steps:
(i) Soaked pelt was pre-dehaired in the drum with 100% (% is weight percentage based on the weight of the salted pelt) of water at 26°C, with 0.20% of enzymes (Bemanol RS-300; obtainable from Stahl Europe BV), 0.50% of reducing agent (Bemanol RS-200; obtainable from Stahl Europe BV), followed by adding 0.20% of sodium sulphide to start the further dehairing process. The mixture was ran for 45 minutes.
(ii) 0.05% of Eusapon EM (a wetting agent obtainable from Stahl Europe BV) and 0.10% of Eusapon OC (a degreasing agent obtainable from Stahl Europe BV) and 2.0% sodium metasilicate were added. The mixture was ran for 45 minutes.
(iii) Dehairing was initiated by adding 0.80% of sodium hydrosulphide and 0.60% of sodium sulphide. After running for 120 minutes, the immunized hair was removed from the bath by filtration. Next, 2.0% sodium metasilicate was added. The mixture was run for 120 minutes.
(iv) Liming was initiated by adding 0.50% sodium hydroxide, 0.05% of enzymes (Bemanol RS-300; obtainable from Stahl Europe BV), 0.50% of sodium sulphide and 0.30% of reducing agent (Bemanol RS-200; obtainable from Stahl Europe BV). The mixture was left overnight on a program, running 5 minutes and stopping 25 minutes consecutively.
(v) The pelts were removed from the drums and fleshed and splitted.

Thereafter, using conventional methods, crust was obtained after deliming (actually neutralization step), bating, pickling, chrome tanning, retanning, neutralization, dyeing and fatliquoring.

### Example 8 - comparison leathers

The leather obtained from Example 1 to 5 and Comparative Example 6 and 7 were visually and tactilely assessed for fullness, tightness, openness and colour brightness. The leathers were graded for these properties combined on a scale from 1 to 10. The higher the number attributed the better.

| Example | Grade |
|---|---|
| Example 1 (ratio 90/10) | 6.5 |
| Example 2 (ratio 85/15) | 8.5 |
| Comparative Example 3 (ratio 80/20) | 9.0 |
| Example 4 (ratio 75/25) | 6.5 |
| Example 5 (ratio 70/30) | 5.0 |
| Comparative Example 6 (lime) | 6.0 |
| Comparative Example 6 (only silicate) | 4.0 |

The quality of the leathers made according to Examples 1 to 4 were graded as better than the Comparative Examples 6 and 7. The quality of the leather of Comparative Example 7 was graded lowest. The quality of the leather of Example 2 and comparative Example 3 were graded highest.

### Example 9 - Composition of effluent

The effluent from the Examples 1-3 and Comparative Example 6 (lime) was evaluated. The COD (chemical oxygen demand), BOD₅ (biological oxygen demand in 5 days), Total Nitrogen (TN), sulphide content (S²⁻) and TDS (total dissolved ionisable solids) and TSS (total suspended solids) were measured.

COD was measured according to ISO 15705. A lower COD value, expressed in mg per kg of skin/pelt, is better. BOD₅ was measured according to EN 1899-1. The ratio BOD₅/COD was calculated from the measured values of COD and BOD₅. A BOD₅/COD ratio of between 0.3 and 0.6 is seen as optimum, because this means that the effluent is better biodegradable in a wastewater treatment facility. TN was measured according to ISO 11905-1. A lower TN value, expressed in mg per kg of skin/pelt, is better. S²⁻ was measured according to ISO 10530-1991. A lower S²⁻ value, expressed in mg per kg of skin/pelt, is better. TDS was measured via conductivity. Dissolved ionized solids, such as salts and minerals, increase the electrical conductivity (EC) of a solution. Because it is a volume measure of ionized solids, EC can be used to estimate TDS. Dissolved organic solids, such as sugar, and microscopic solid particles, such as colloids, do not significantly affect the conductivity of a solution, and are not taken into account. A lower TDS value, expressed in mg per kg of skin/pelt, is better. TSS (total suspended solids) is measured by weight. A glass fiber filter disc, of which the empty weight was determined, is used as a filter in a filtering flask. A defined sample volume of effluent is filtered over this filter and the residue, on the same fiber filter disc, is dried to a constant weight in an oven at 105°C. The weight difference is the Total Suspended Solids. According to the volume used, the suspended solids can be expressed as mg/L, which can be recalculated to mg per kg of skin/pelt. The method is according to ISO 11923:1997. A lower TSS value, expressed in mg per kg of skin/pelt, is desired.

| From Example | COD | BOD₅ | BOD₅/COD | TN | TDS | TSS | S²⁻ |
|---|---|---|---|---|---|---|---|
| Example 1 | 103725 | 49601 | 0,48 | 5785 | 60756 | | 835 |
| Example 2 | 74368 | 28119 | 0,38 | 3796 | 53430 | 6428 | 295 |
| C. Example 3 | 83207 | 31283 | 0,38 | 3441 | 48872 | | 759 |
| C. Example 6 | 85930 | 27888 | 0,32 | 5596 | 51744 | 30729 | 1142 |

The COD of Example 2 was notably lower than that of Comparative Example 6, but the COD of Example 1 was higher. The BOD₅/COD ratio for Examples 1-3 were all higher and closer to optimum level, which would be 0.6, than for Comparative Example 6. The TN of Examples 2 and 3 was notably lower than of Comparative Example 6. The S²⁻ of Examples 1-3 was notably lower than of Comparative Example 6. Taking into account COD, BOD₅/COD, TN, TDS and S²⁻, the effluents of Examples 2 and 3 are considered the least polluted and thus best. The TSS was measured only of Example 2 and Comparative Example 6. The TSS was much lower for Example 2 than for Comparative Example 6.

## Claims

1. A process for dehairing and/or liming of hides, skins or pelts comprising treating the hides, skins or pelts with a metal silicate and a strong alkaline in an aqueous composition in the substantial absence of calcium hydroxide meaning that the amount of calcium hydroxide in the aqueous composition is less than 0.2 weight% and wherein the amounts of metal silicate and strong alkaline combined are in the range of 0.2% to 8% per kg of hide, skin or pelt and wherein the ratio of the metal silicate and the strong alkaline is in the range of 90/10 to 75/25 based on weight of both solid materials and where the major component is the metal silicate

2. Process according to claim 1, wherein the strong alkaline is a metal hydroxide such as sodium hydroxide, potassium hydroxide or lithium hydroxide.

3. Process according to claim 1 or 2, wherein the metal silicate is sodium metasilicate, sodium orthosilicate, either individually or in any combination.

4. Process according to any one of claims 1 to 3, wherein the aqueous composition for treating the hides, skins or pelts also comprises a protein disulphide reducing compound.

5. Process according to claim 4, wherein the protein disulphide reducing compound is one or a combination of substances selected from the group consisting of sodium hydrosulphide, sodium sulphide, potassium sulphide or lithium sulphide, salts of thioglycolic acid, or other thiols (mercaptans) or enzymes capable of catalysing the rearrangement of disulphide bonds in protein.

6. Process according to any one of claims 1 to 5, wherein the amounts of metal silicate and alkaline combined are in the range of 0.5% to 4%, preferably from 1% to 2.5% per kg of hide, skin or pelt.

7. Process according to any one of claims 1 to 6, wherein the ratio of metal silicate and alkaline is in the range of 90/10 to 80/20, preferably in the range 85/15 to 80/20, where the ratio is based on weight of both solid materials and where the major component is the metal silicate.

8. Process according to any one of claims 1 to 7, comprising the steps:
i) optionally, treating the soaked hide/skin with 0.05-0.5% of enzymes, 0.01-0.1% of probiotic auxiliaries, 0.05-0.3% of reducing agent and 0.10-1.0% of a mercaptan product at a temperature of 15-30°C in the presence of 50-150% of water, preferably under stirring condition;
ii) adding a metal silicate and an alkaline and treating the hide/skin/pelt for a period of between 10 minutes and 1 hour, optionally in the presence of a probiotic agent, degreasing agent or wetting agent;
iii) adding protein disulphide reducing agents, such as 0.2-3.5% of sodium hydrosulphide and/or 0.1-3.0% sodium sulphide, and continuing the treatment for between 2 hours and 8 hours, after or during which the immunized hair is removed by filtration;
iv) subsequently adding water, metal silicate and an alkaline and optionally a further amount of one or more protein disulphide reducing agents, such as 0.1-1.0% of sodium sulphide, and continuing the treatment for between 3 hours and 20 hours;
(v) optionally, adding 0.1-1.0% of an oxidizer to remove residual sulphide present in the float.

9. A dehairing and/or liming composition comprising a metal silicate and a strong alkaline and substantially free of calcium hydroxide meaning that the amount of calcium hydroxide in the composition is less than 0.2 weight% and wherein the amounts of metal silicate and alkaline combined are in the range of 0.2% to 8% per kg of hide, skin or pelt and wherein the ratio of metal silicate and alkaline is in the range of 90/10 to 75/25, preferably in the range of 90/10 to 80/20, more preferably 85/15 to 80/20 where the ratio is based on weight of both solid materials and where the major component is the metal silicate.

10. A dehairing and/or liming composition according to claim 9, wherein the metal silicate is sodium metasilicate, water glass, sodium orthosilicate, either individually or in any combination.

11. The use of a dehairing and/or liming composition as defined in any one of claims 9 or 10 to treat hides, skins or pelts.

12. Dehaired hides, skins or pelts obtainable by the process as defined in any of claims 1 to 8.

13. Process for preparing crust or leather from the dehaired hides, skins or pelts as defined in claim 12 comprising the following steps: deliming (actually neutralization step), bating, pickling, chrome tanning, retanning, neutralization, dyeing and fatliquoring.

14. Crust or leather obtainable by the process as defined in claim 13.

## Patentansprüche

1. Verfahren zum Enthaaren und/oder Kälken von Häuten, Fellen und Pelzen, umfassend das Behandeln der Häute, Felle und Pelze mit einem Metallsilikat und einem starken Alkali in einer wässrigen Zusammensetzung, im Wesentlichen frei von Calciumhydroxid, was bedeutet, dass die Menge an Calciumhydroxid in der wässrigen Zusammensetzung weniger als 0,2 Gew.-% beträgt und wobei die Mengen an Metallsilikat und starkem Alkali zusammen im Bereich von 0,2 % bis 8 % pro kg Haut, Fell und Pelz sind und wobei das Verhältnis von Metallsilikat und starkem Alkali im Bereich von 90/10 bis 75/25 ist, basierend auf dem Gewicht der beiden festen Materialien, und wo der Hauptbestandteil das Metallsilikat ist.

2. Verfahren nach Anspruch 1, wobei das starke Alkali ein Metallhydroxid wie Natriumhydroxid, Kaliumhydroxid oder Lithiumhydroxid ist.

3. Verfahren nach Anspruch 1 oder 2, wobei das Metallsilikat Natriummetasilikat, Natriumorthosilikat, entweder einzeln oder in beliebiger Kombination, ist.

4. Verfahren nach einem der Ansprüche 1 bis 3, wobei die wässrige Zusammensetzung zur Behandlung der Häute, Felle und Pelze auch eine Protein-Disulfid-reduzierende Verbindung umfasst.

5. Verfahren nach Anspruch 4, wobei die Protein-Disulfid-reduzierende Verbindung eine oder eine Kombination von Substanzen ist, die ausgewählt sind aus der Gruppe bestehend aus Natriumhydrosulfid, Natriumsulfid, Kaliumsulfid oder Lithiumsulfid, Salzen der Thioglycolsäure oder anderen Thiolen (Mercaptanen) oder Enzymen, geeignet, die Umlagerung von Disulfidbindungen in Proteinen zu katalysieren.

6. Verfahren nach einem der Ansprüche 1 bis 5, wobei die Mengen an Metallsilikat und Alkali zusammen im Bereich von 0,5 % bis 4 %, vorzugsweise von 1 % bis 2,5 % pro kg Haut, Fell und Pelz, sind.

7. Verfahren nach einem der Ansprüche 1 bis 6, wobei das Verhältnis von Metallsilikat und Alkali im Bereich von 90/10 bis 80/20, vorzugsweise im Bereich von 85/15 bis 80/20 ist, wobei das Verhältnis auf dem Gewicht beider Feststoffe basiert und wobei der Hauptbestandteil das Metallsilikat ist.

8. Verfahren nach einem der Ansprüche 1 bis 7, umfassend die Schritte:
i) optional Behandeln der/s eingeweichten Haut/Fell mit 0,05 - 0,5 % Enzymen, 0,01 - 0,1 % probiotischen Hilfsstoffen, 0,05 - 0,3 % Reduktionsmittel und 0,10 - 1,0 % eines Mercaptanprodukts bei einer Temperatur von 15 - 30 °C in Gegenwart von 50 - 150 % Wasser, vorzugsweise unter Rühren,
ii) Zugeben eines Metallsilikats und eines Alkalis und Behandeln der/s Haut, Fells und Pelzes für einen Zeitraum von zwischen 10 Minuten und 1 Stunde, optional in Gegenwart eines probiotischen Mittels, Entfettungsmittels oder Benetzungsmittels;
iii) Zugeben von Protein-Disulfid reduzierenden Mitteln, wie z. B. 0,2 - 3,5 % Natriumhydrosulfid und/oder 0,1 - 3,0 % Natriumsulfid, und Fortsetzen der Behandlung für zwischen 2 Stunden bis 8 Stunden, wonach oder währenddessen das immunisierte Haar durch Filtration entfernt wird;
iv) anschließendes Zugeben von Wasser, Metallsilikat und einer alkalischen und optional einer weiteren Menge eines oder mehrerer Protein-Disulfid reduzierender Mittel, wie z. B. 0,1 - 1,0 % Natriumsulfid, und Fortsetzen der Behandlung für zwischen 3 Stunden und 20 Stunden;
v) optional Zugeben von 0,1 - 1,0 % eines Oxidationsmittels, um im Schwimmer vorhandenes Restsulfid zu entfernen.

9. Enthaarungs- und/oder Kälkungszusammensetzung, umfassend ein Metallsilikat und ein starkes Alkali und im Wesentlichen frei von Calciumhydroxid, was bedeutet, dass die Menge an Calciumhydroxid in der Zusammensetzung weniger als 0,2 Gew.-% beträgt und wobei die Mengen an Metallsilikat und Alkali zusammen im Bereich von 0,2 % bis 8 % pro kg Haut, Fell und Pelz sind und wobei das Verhältnis von Metallsilikat und Alkali im Bereich von 90/10 bis 75/25, vorzugsweise im Bereich von 90/10 bis 80/20, stärker bevorzugt 85/15 bis 80/20 ist, wobei das Verhältnis auf dem Gewicht beider Feststoffe basiert und wobei der Hauptbestandteil das Metallsilikat ist.

10. Enthaarungs- und/oder Kälkungszusammensetzung gemäß Anspruch 9, wobei das Metallsilikat Natriummetasilikat, Wasserglas, Natriumorthosilikat, entweder einzeln oder in beliebiger Kombination, ist.

11. Verwendung einer Enthaarungs- und/oder Kälkungszusammensetzung wie in einem der Ansprüche 9 oder 10 definiert zur Behandlung von Häuten, Fellen und Pelzen.

12. Enthaarte Häute, Felle und Pelze, erhältlich nach einem der in den Ansprüchen 1 bis 8 definierten Verfahren.

13. Verfahren zum Herstellen von Kruste oder Leder aus den enthaarten Häuten, Fellen und Pelzen nach Anspruch 12, umfassend die folgenden Schritte: Entkälken (tatsächlich ein Neutralisierungsschritt), Beizen, Pickeln, Chromgerben, Nachgerben, Neutralisieren, Färben und Fetten.

14. Kruste oder Leder, erhältlich durch das in Anspruch 13 definierte Verfahren.

## Revendications

1. Un procédé d'épilation et/ou de chaulage de cuirs, peaux ou fourrures comprenant le traitement des cuirs, peaux ou fourrures par un silicate métallique et un composé alcalin fort dans une composition aqueuse en l'absence substantielle d'hydroxyde de calcium, signifiant que la quantité d'hydroxyde de calcium dans la composition aqueuse est inférieure à 0,2 % en poids et dans lequel les quantités de silicate métallique et de composé alcalin fort combinées sont dans la plage de 0,2% à 8% par kg de cuir, peau ou fourrure et dans lequel le rapport silicate métallique/composé alcalin fort est dans la plage de 90/10 à 75/25 sur la base du poids des deux matières solides et où le composant majoritaire est le silicate métallique.

2. Procédé selon la revendication 1, dans lequel l'alcalin fort est un hydroxyde métallique tel que l'hydroxyde de sodium, l'hydroxyde de potassium ou l'hydroxyde de lithium.

3. Procédé selon la revendication 1 ou 2, dans lequel le silicate métallique est le métasilicate de sodium, l'orthosilicate de sodium, soit individuellement, soit en combinaison.

4. Procédé selon l'une quelconque des revendications 1 à 3, dans lequel la composition aqueuse destinée au traitement des cuirs, peaux ou fourrures comprend également un composé réducteur consistant en disulfure de protéines.

5. Procédé selon la revendication 4, dans lequel le composé réducteur de disulfure de protéines est individuellement ou en combinaison avec des substances choisies dans le groupe constitué par l'hydrosulfure de sodium, le sulfure de sodium, le sulfure de potassium ou le sulfure de lithium, les sels de l'acide thioglycolique, ou autres thiols (mercaptans) ou enzymes capables de catalyser le réarrangement des liaisons disulfures dans les protéines.

6. Procédé selon l'une quelconque des revendications 1 à 5, dans lequel les quantités de silicate métallique et de composés alcalins combinées sont comprises dans la gamme de 0,5% à 4%, de préférence de 1% à 2,5% par kg de cuir, de peau ou de fourrure.

7. Procédé selon l'une quelconque des revendications 1 à 6, dans lequel le rapport silicate métallique/ et du composé alcalin est compris entre 90/10 et 80/20, de préférence entre 85/15 et 80/20, où le rapport est basé sur le poids des deux matières solides et où le composant majoritaire est le silicate métallique.

8. Procédé selon l'une quelconque des revendications 1 à 7, comprenant les étapes suivantes :
i) éventuellement, le traitement des cuirs/peaux trempés par 0,05 - 0,5 % d'enzymes, 0,01- 0,1 % d'auxiliaires probiotiques, 0,05 - 0,3 % d'agent réducteur et 0,10 - 1,0 % d'un mercaptan produit à une température de 15 - 30°C en présence de 50 - 150 % d'eau, de préférence sous agitation ;
ii) ajouter un silicate métallique et un composé alcalin et traiter cuir/peau/fourrure pendant une période comprise entre 10 minutes et 1 heure, éventuellement en présence d'un agent probiotique, d'un agent dégraissant ou d'un agent mouillant ;
iii) ajouter des agents réducteurs de disulfure de protéines, tels que 0,2 à 3,5 % d'hydrosulfure de sodium et/ou 0,1 à 3,0 % de sulfure de sodium, et poursuivre le traitement pendant une période comprise entre 2 heures et 8 heures, après ou pendant laquelle les poils immunisés sont éliminés par filtration ;
iv) ajouter ensuite de l'eau, du silicate métallique et un composé alcalin et éventuellement une quantité supplémentaire d'un ou plusieurs agents réducteurs de disulfure de protéines, tels que 0,1 à 1,0 % de sulfure de sodium, et poursuivre le traitement pendant une période comprise entre 3 heures et 20 heures ;
v) éventuellement, ajouter 0,1 à 1,0 % d'un oxydant pour éliminer le sulfure résiduel présent dans le flotté.

9. Une composition d'épilage et/ou de chaulage comprenant un silicate métallique et un composé alcalin fort et sensiblement exempt d'hydroxyde de calcium, signifiant que la quantité d'hydroxyde de calcium dans la composition est inférieure à 0,2 % en poids et dans laquelle les quantités de silicate métallique et d'alcalin combinées sont comprises entre 0,2 % et 8 % par kg de cuir, de peau ou de fourrure et dans laquelle le rapport de silicate métallique et de composé alcalin est compris entre 90/10 et 75/25, de préférence entre 90/10 et 80/20, plus préférablement entre 85/15 et 80/20, le rapport étant basé sur le poids des deux matières solides et le composant majoritaire étant le silicate métallique.

10. Une composition d'épilage et/ou de chaulage selon la revendication 9, dans laquelle le silicate métallique est le métasilicate de sodium, silicate de sodium, l'orthosilicate de sodium, soit individuellement, soit dans n'importe quelle combinaison.

11. L'utilisation d'une composition d'épilage et/ou de chaulage telle que définie dans l'une quelconque des revendications 9 ou 10 pour traiter des cuirs, peaux ou fourrures.

12. Cuirs, peaux ou fourrures épilés pouvant être obtenus par le procédé tel que défini dans l'une quelconque des revendications 1 à 8.

13. Procédé de préparation de croûte ou de cuir à partir de cuirs, peaux ou fourrures épilés tels que définis dans la revendication 12 comprenant les étapes suivantes : déchaulage (en fait étape de neutralisation), raffinage, décapage, tannage au chrome, retannage, neutralisation, teinture et engraissage.

14. Cuir en croûte ou cuir pouvant être obtenu par le procédé tel que défini dans la revendication 13.
